# EUROPEAN PATENT SPECIFICATION

(11) **EP 1 578 214 B1**
(45) Date of publication and mention of the grant of the patent: **27.05.2009**
(21) Application number: 03786477.4
(22) Date of filing: 21.12.2003
(51) Int. Cl.: A23L 1/29, A61K 31/23

(54) **FOOD COMPOSITION COMPRISING A SATIETY ENHANCING AMOUNT OF TRIACYLGLYCEROL**
LEBENSMITTELZUSAMMENSETZUNG MIT EINEM SÄTTIGUNGSVERSTÄRKENDEN TRIACYLGLYCERINANTEIL
COMPOSITION ALIMENTAIRE COMPRENANT UNE QUANTITE DE TRIACYLGLYCEROL AMELIORANT LA SATIETE

(30) Priority: 27.12.2002 SE 0203886
(43) Date of publication of application: 28.09.2005
(73) Proprietor: DSM IP Assets B.V., 6411 TE Heerlen (NL)
(72) Inventor: HERSLÖF, Bengt, S-11421 Stockholm (SE); VIBERG, Annika, S-17547 Järfälla (SE)
(74) Representative: van Grieken-Plooster, Izabella Johanna
(86) International application number: PCT/SE2003/002054
(87) International publication number: WO 2004/057982

(56) References cited:
- EP-A1- 0 225 303
- WO-A-99/02041
- WO-A2-02/00042
- US-A- 5 258 197

## Description

### FIELD OF THE INVENTION

The present invention relates to a glycerol ester product which is useful as a food additive for satiety control, a method for satiety control comprising administration of the glycerol ester product to a person in need thereof, and a food composition comprising the glycerol ester product.

### BACKGROUND OF THE INVENTION

Excess energy intake by over-consumption of food is considered a major health problem, in particular in the Western world. Various methods and food products that counter such intake are known in the art. One particular kind of energy intake reducing products is based on the concept that food products with a high fat content bring about a feeling of satiety soon after ingestion. On the other hand, such food products are rich in calories and thus should only be ingested in comparatively small amounts. Thus a delicate balance between satiety effect onset and energy intake necessary to achieve that effect has to be stricken.

WO 99/02041 discloses a food composition giving a prolonged feeling of satiety comprising a mixture of triglyceride oils, in particular a oil-in-water emulsion thereof, having a solid fat content at ambient to body temperature and a food emulsifier, such as lecithin, galactolipids and fractionated oat oil. While the food composition of WO 99/02041 has been shown to provide the desired effect in practice, the energy intake by the body due to its consumption is of a size which far from optimal. Thus there is room for improvement in regard of satiety control products based on fat.

EP 0 225 303 A1 describes the use of a hydrophobic substance for the treatment of obesity. This hydrophobic substance can be a glycerol ester of fatty acids. However, use of this hydrophobic substance is connected with the same limitations as the food composition of WO 99/02041.

The same holds for US 5,258,197 disclosing fat mixtures as low calorie fat wherein the triglycerides comprise long chain fatty acid residues and short chain residues.

WO 02/00042 A2 dicloses oral compositions consisting of long chain fatty acids and esters thereof wherein the esters are explicity described as non-glycerol esters.

### OBJECTS OF THE INVENTION

It is an object of the present invention to provide a satiety control product of the aforementioned kind, which is improved in regard of the energy intake necessary to obtain a desired satiety feeling.

It is another object of the invention to provide a method of satiety control by use of said satiety control product.

Further objects of the invention will become evident from the study of the following summary of the invention, the description of a preferred embodiment thereof, and the appended claims.

### SUMMARY OF THE INVENTION

The invention is based on the hypothesis that the hydrolysis of triacylglycerols in the gastrointestinal tract by the action of lipases will be retarded if one or several of the acyl rests are substituted in α-position by an alkyl rest, and that this will delay the onset of hunger, thus leading to a reduced energy intake during a subsequent meal which is consumed within the span of time in which the appetite is suppressed, such as up to eight hours and more.

According to the present invention is disclosed a satiety control product comprising an satiety enhancing amount of a triacylglycerol or a mixture of triacylglycerols the acyl residues of which are selected from the group consisting of tetradecanoyl to eicosanoyl, optionally mono- or di-unsaturated, optionally α-monosubstituted with saturated C₁-C₄ alkyl, with the proviso that more than 15 mole-% of total acyl residues are comprised by α-alkyl substituted acyl residues wherein the α-alkyl substituents comprise up to 9 carbon atoms, and a food emulsifier.

Preferred triacylglycerols according to the invention are those which comprise hexadecanoyl and/or octadecanoyl residues and corresponding acyl residues alkylated, in particular, methylated in α-position. The distribution of the acyl residues between the glycerol entities may be statistic or given. In other words, for instance, 1-(2-methylhexadecanoyl)-2,3-dioctadecanoyl glycerol ("ordered") and the "statistic" mixture of (2-methylhecadecanoyl), (octadecanoyl) glycerols comprising these acyl rests in a 1:2 molar ratio, obtained by, for instance, from of the a corresponding ordered compound by acid or alkali catalysed trans-esterification, are both comprised by the invention.

Preferred α**-**alkyl substituents are C₁-C₄ saturated alkyl, in particular methyl, ethyl, propyl, isoproyl, butyl, isobutyl but, exceptionally, alkyl groups comprising up to 9 carbon atoms may be used. Most preferred is methyl, ethyl, propyl as α-substituent.

Thus, in particular, according to the invention is disclosed a food composition comprising a satiety enhancing amount of a triacylglycerol or a mixture of triacylglycerols, the acyl residues of which are selected from the group consisting of tetradecanoyl to eicosanoyl, optionally mono- or di-unsaturated, optionally α-monosubstituted with saturated C₁-C₄ alkyl, with the proviso that more than 15 mole-% of total acyl residues are comprised by α-alkyl substituted acyl residues wherein the α-alkyl substituents comprise up to 9 carbon atoms, and a food emulsifier. Preferably more than 50 mole-%, more preferred more than 80 mole-%, even more preferred more than 90 mole-%, most preferred more than 95 mole-%, of said α-substituted acyl residues are comprised by a member of the group consisting of 2-methyltetradecanoyl, 2-methylhexadecanoyl, 2-methyloctadecanoyl, 2-methyleicosanoyl, and mixtures thereof.

According to a preferred aspect of the invention the emulsifier comprises lecithin, and/or galactolipid. It is preferred for the galactolipid to be comprised by fractionated oat oil.

According to first preferred aspect the food composition of the invention is an oil-in-water emulsion.

The food composition of the invention is particularly suitable for the treatment or prevention of obesity.

According to a second preferred aspect of the invention is disclosed a food additive essentially consisting of a triacylglycerol or a mixture of triacylglycerols, the acyl residues of which are selected from the group consisting of tetradecanoyl to eicosanoyl, optionally mono- or di-unsaturated, optionally α-monosubstituted with saturated C₁-C₄ alkyl, with the proviso that more than 15 mole-% of total acyl residues are comprised by α-alkyl substituted acyl residues wherein the α-alkyl substituents comprise up to 9 carbon atoms, a food emulsifier and, optionally, one or several of water, sodium chloride, antioxidant, colorant, flavouring. The food emulsifier is preferably selected from lecithin, galactolipid, and their mixtures.

According to a third preferred aspect of the invention is disclosed a food product comprising an obesity-preventing amount of the aforementioned food additive. The food product may be in form of a dairy product, in particular in form of yoghurt, cream, sour cream, milk, butter or a butter substitute comprising milk fat, ice cream. The food product of the invention may however also be in form of a non-dairy product.

According to a fourth preferred aspect of the invention is disclosed a method of controlling satiety in a person, comprising:
- providing to said person a food product comprising a satiety controlling amount of a food additive essentially consisting of a triacylglycerol or a mixture of triacylglycerols, the acyl residues of which are selected from the group consisting of tetradecanoyl to eicosanoyl, optionally mono- or di-unsaturated, optionally α-monosubstituted with saturated C₁-C₄ alkyl, with the proviso that more than 15 mole-% of total acyl residues are comprised by α-substituted acyl residues, a food emulsifier and, optionally, one or several of water, sodium chloride, antioxidant, colorant, flavouring; and
- directing said person to consume said food product from 1 h to 8 h prior to a meal selected from lunch, dinner, supper,
thereby reducing the amount of food consumed in the meal in comparison with the amount of food consumed in a corresponding meal following, by a corresponding time difference, the consumption of a food product of equal energy content but lacking said food additive. It is preferred that more than 50 mole-%, in particular more than 80 mol-%, more particularly more than 90 mol-%, most preferred more than 95 mole-%, of the triacylglycerol mixture are 1-(2-methyloctadecanoyl)-2,3-dihexadecanoyl-glycerol. Also preferred is for about a third of the acyl residues of the triacylglycerol mixture to be octadecanoyl and for about two thirds to be 1-(2-methyloctadecanoyl).

### DESCRIPTION OF A PREFERRED EMBODIMENT

**Materials.** A mixture of (hexadecanoyl)ₓ-(1-methyloctadecanoyl)_{y}-glycerols in which x + y = 3 was obtained by trans-esterification of corresponding molar amounts of tripalmitin and tri-(2-methylstearoyl)glycerol in n-hexane with sodium methoxide as catalyst. The reaction was carried out in an inert atmosphere. The reaction product was washed with water and hexane; it may be purified by chromatography on silica gel with 2-propanol and hexane. It is a white powder essentially consisting of an isomer mixture of tripalmitin, tri(2-methylstearoyl)glycerol, 1,2-di(2-methyl-stearoyl)-3-palmitoylglycerol, 1,3-di(2-methyl-stearoyl)-2-palmitoylglycerol, 1-(2-methylstearoyl)-2,3-dipalmitoyl-glycerol, and 1,3-dipalmitoyl-2-(2-methylstearoyl)glycerol.

Tri(2-methylstearoyl)glycerol was prepared by acylating glycerol in the presence of pyridine with 2-methyloctadecanoyl chloride prepared from 2-methyloctadecanoic acid and thionyl chloride.

1-(2-methyloctadecanoyl)-2,3-dihexadecanoyl-glycerol was prepared from 1-(2-methyloctadecanoyl)-2,3-isopropylidene glycerol (Gronowitz, S. et al., Lipids 32:6 (1997) 6667-673) by reaction with 2 moles of 2-methyloctadecanoyl chloride in the presence of pyridine according to *Procedure B, ibid.,* p. 667. Other triacylglycerols suitable in the invention, such as tris(2-methyloctadecanoyl)glycerol, are disclosed in Gronowitz et al. or can be prepared by adapting the methods of Gronowitz et al. in a manner which is within the easy reach of the person skilled in the art.

### EXAMPLE 1. Preparation of triglyceride test emulsions.

The following oil-in-water test emulsions containing 20% by weight of triglyceride were prepared by mixing the components listed in Table 1 with distilled water using a high-speed stirrer.

**Table 1. Composition of test emulsions, in % by weight, rest water**

| Test Emulsion | Fractionated Palm Oil | Fractionated Oat Oil | Triacylglycerols of the Invention |
|---|---|---|---|
| Prior Art (A) | 40 | 2.5 | 0 |
| Invention (B) | 0 | 2.5 | 10 |

### EXAMPLE 2. Preparation of test yoghurt.

Yoghurt products for testing were prepared ex tempore by mixing equal amounts of the test emulsions with a commercial low fat yoghurt (Lätt-Yoggi; Arla, Stockholm, Sweden). The fat content of the test yoghurts was normalized to 3% by weight obtained by adding double cream to the low fat products to compensate for differences in energy content. The composition of the test yoghurts is shown in Table 2.

**Table 2. Composition of test yoghurts**

| Test emulsion | Amount of Yoghurt (g) | Amount of Test Emulsion Added (g) | Amount of Double Cream Added (g) | Total Fat Content (g) |
|---|---|---|---|---|
| Prior Art (A) | 187.5 | 12.5 | 0 | 6 |
| Invention (B) | 187.5 | 2.5 | 11.75 | 6 |
| Control | 187.5 | 0 | 12.5 | 6 |

### EXAMPLE 3. Mean intake at lunch following yoghurt breakfast

Twelve non-overweight healthy volunteers (six men and six women) were given 200 g yoghurt for breakfast at 8 o'clock in the morning. At noon the volunteers were served a test meal (lunch) at which they had free access to a wide range of familiar foods. For each person, all test meal foods were weighed prior to the meal, and all uneaten foods was weighed after the meal. Intake was assessed by difference in weight of food. The tests were performed on three days separated by an interval of one week. The study was of a single-blind randomised crossover design. The test persons were asked to fast from 8 o'clock in the evening of the preceding day and to refrain from heavy exercise on that day and on the day when the test was performed. They were instructed to not consumed anything except non-carbonated water between the test breakfast and the test lunch. The results of the test are given in Table 3.

**Table 3. Mean energy intake at test lunch**

| Volunteers | Energy Intake, Yoghurt (Control) | Energy Intake, Yoghurt + B | Energy Consumption, Yoghurt + B | Energy Intake, Yoghurt + C | Energy Consumption, Yoghurt + C |
|---|---|---|---|---|---|
| Male | 7.01 | 5.57 | -20.5 | 5.24 | -25.2 |
| Female | 5.12 | 3.89 | -24.1 | 3.63 | -29.2 |
| Total | 6.07 | 4.71 | -22.3 | 4.41 | -27.2 |

The energy intake at the subsequent meal (test lunch) was reduced for all subjects that had consumed the satiety control product containing the prior art and the inventive test emulsion, respectively. The efficacy of the test emulsion of the invention (C) was however found to be more than ten times higher than that of the prior art emulsion (B).

## Claims

1. A composition, in particular a food composition comprising a satiety enhancing amount of a triacylglycerol or a mixture of triacylglycerols, the acyl residues of which are selected from the group consisting of tetradecanoyl to eicosanoyl, optionally mono- or di-unsaturated, with the proviso that more than 15 mole-% of total acyl residues are comprised by α-alkyl substituted acyl residues wherein the α-alkyl substituents comprise up to 9 carbon atoms, and a food emulsifier.

2. The composition of claim 1, wherein more than 50 mole-% of said α-substituted acyl residues are comprised by a member of the group consisting of 2-methyltetradecanoyl, 2-methylhexadecanoyl, 2-methyloctadecanoyl, 2-methyleicosanoyl, and mixtures thereof.

3. The composition of claim 1, wherein more than 80 mole-% of said α-substituted acyl residues are comprised by a member of the group consisting of 2-methyltetradecanoyl, 2-methylhexadecanoyl, 2-methyloctadecanoyl, 2-methyleicosanoyl, and mixtures thereof.

4. The composition of claim 1, wherein more than 90 mole-% of said α-substituted acyl residues are comprised by a member of the group consisting of 2-methyltetradecanoyl, 2-methylhexadecanoyl, 2-methyloctadecanoyl, 2-methyleicosanoyl, and mixtures thereof.

5. The composition of claim 1, wherein more than 95 mole-% of said α-substituted acyl residues are comprised by a member of the group consisting of 2-methyltetradecanoyl, 2-methylhexadecanoyl, 2-methyloctadecanoyl, 2-methyleicosanoyl, and mixtures thereof.

6. The composition of any of claims 1 to 5, wherein said α-substituted acyl residue is 2-methyloctadecanoyl.

7. The composition of any of claim 6, wherein said acyl residues that are not α-substituted are selected from hexadecanoyl and octadecanoyl.

8. The composition of claim 1, wherein said acyl residues comprise hexadecanoyl and 2-methyloctadecanoyl, with the proviso that 15 mole % or more of the acyl residues are 2-methyloctadecanoyl residues.

9. The composition of claim 8, wherein said acyl residues essentially consist of hexadecanoyl and 2-methyloctadecanoyl.

10. The composition of claim 8 or 9, wherein the molar ratio of hexadecanoyl to 2-methyloctadecanoyl is about 1:2.

11. The composition of claim 1 essentially consisting of tripalmitin, tri(2-methylstearoyl)glycerol, 1,2-di(2-methyl-stearoyl)-3-palmitoylglycerol, 1,3-di(2-methyl-stearoyl)-2-palmitoylglycerol, 1-(2-methylstearoyl)-2,3-dipalmitoyl-glycerol, and 1,3-dipalmitoyl-2-(2-methylstearoyl)glycerol.

12. The composition of claim 1, wherein the food emulsifier comprises lecithin.

13. The composition of claim 1, wherein the food emulsifier comprises galactolipid.

14. The composition of claim 13, wherein the galactolipid is comprised by fractionated oat oil.

15. The composition of claim 14, wherein the amount of fractionated oat oil is about 1.5% by weight of the total composition.

16. The composition of claim 1, in form of an oil-in-water emulsion.

17. The composition of any of claims 1 to 16 for use in therapy.

18. Use of the composition of any of claims 1 to 16 in the manufacture of a medicament for the treatment or prevention of obesity.

19. Food additive essentially consisting of a triacylglycerol or a mixture of triacylglycerols, the acyl residues of which are selected from the group consisting of tetradecanoyl to eicosanoyl, optionally mono- or di-unsaturated, with the proviso that more than 15 mole-% of total acyl residues are comprised by α-alkyl substituted acyl residues wherein the α-alkyl substituents comprise up to 9 carbon atoms, a food emulsifier and, optionally, one or several of water, sodium chloride, antioxidant, colorant, flavouring.

20. The food additive of claim 19, wherein said food emulsifier is selected from lecithin and galactolipid.

21. A food product comprising an obesity preventing amount of the food additive of claim 19 or 20.

22. The food product of claim 21 in form of a dairy product.

23. The dairy product of claim 22 in form of yoghurt, cream, sour cream, milk, butter or a butter substitute comprising milk fat, ice cream.

24. The food product of claim 21, in form of a non-dairy product.

25. The food product of claim 21, wherein more than 50 mole-% of said triacylglycerol mixture is 1-(2-methyloctadecanoyl)-2,3-dihexadecanoyl-glycerol.

26. The food product of claim 21, wherein about a third of said acyl residues are octadecanoyl and about two thirds are 1-(2-methyloctadecanoyl).

## Patentansprüche

1. Zusammensetzung, insbesondere eine Nahrungsmittelzusammensetzung, umfassend eine sättigungsfördernde Menge eines Triacylglycerins oder eines Gemischs von Triacylglycerinen, deren Acylreste ausgewählt sind aus der Gruppe, bestehend aus Tetradecanoyl bis Eicosanoyl (gegebenenfalls einfach oder zweifach ungesättigt), mit der Maßgabe, dass mehr als 15 mol-% aller Acylreste aus α-Alkyl-substituierten Acylresten bestehen, wobei die α-Alkyl-Substituenten bis zu 9 Kohlenstoffatome umfassen, sowie einen Nahrungsmittel-Emulgator.

2. Zusammensetzung gemäß Anspruch 1, wobei mehr als 50 mol-% der α-substituierten Acylreste aus einem Element der Gruppe bestehen, die aus 2-Methyltetradecanoyl, 2-Methylhexadecanoyl, 2-Methyloctadecanoyl, 2-Methyleicosanoyl und Gemischen davon besteht.

3. Zusammensetzung gemäß Anspruch 1, wobei mehr als 80 mol-% der α-substituierten Acylreste aus einem Element der Gruppe bestehen, die aus 2-Methyltetradecanoyl, 2-Methylhexadecanoyl, 2-Methyloctadecanoyl, 2-Methyleicosanoyl und Gemischen davon besteht.

4. Zusammensetzung gemäß Anspruch 1, wobei mehr als 90 mol-% der α-substituierten Acylreste aus einem Element der Gruppe bestehen, die aus 2-Methyltetradecanoyl, 2-Methylhexadecanoyl, 2-Methyloctadecanoyl, 2-Methyleicosanoyl und Gemischen davon besteht.

5. Zusammensetzung gemäß Anspruch 1, wobei mehr als 95 mol-% der α-substituierten Acylreste aus einem Element der Gruppe bestehen, die aus 2-Methyltetradecanoyl, 2-Methylhexadecanoyl, 2-Methyloctadecanoyl, 2-Methyleicosanoyl und Gemischen davon besteht.

6. Zusammensetzung gemäß einem der Ansprüche 1 bis 5, wobei der α-substituierte Acylrest 2-Methyloctadecanoyl ist.

7. Zusammensetzung gemäß einem von Anspruch 6, wobei die Acylreste, die nicht α-substituiert sind, aus Hexadecanoyl und Octadecanoyl ausgewählt sind.

8. Zusammensetzung gemäß Anspruch 1, wobei die Acylreste Hexadecanoyl und 2-Methyloctadecanoyl umfassen, mit der Maßgabe, dass 15 mol-% oder mehr der Acylreste 2-Methyloctadecanoylreste sind.

9. Zusammensetzung gemäß Anspruch 8, wobei die Acylreste im Wesentlichen aus Hexadecanoyl und 2-Methyloctadecanoyl bestehen.

10. Zusammensetzung gemäß Anspruch 8 oder 9, wobei das Molverhältnis von Hexadecanoyl zu 2-Methyloctadecanoyl etwa 1:2 beträgt.

11. Zusammensetzung gemäß Anspruch 1, im Wesentlichen bestehend aus Tripalmitin, Tri(2-methylstearoyl)glycerin, 1,2-Di(2-methylstearoyl)-3-palmitoylglycerin, 1,3-Di(2-methylstearoyl)-2-palmitoylglycerin, 1-(2-Methylstearoyl)-2,3-dipalmitoylglycerin und 1,3-Dipalmitoyl-2-(2-methylstearoyl)glycerin.

12. Zusammensetzung gemäß Anspruch 1, wobei der Nahrungsmittel-Emulgator Lecithin umfasst.

13. Zusammensetzung gemäß Anspruch 1, wobei der Nahrungsmittel-Emulgator Galactolipid umfasst.

14. Zusammensetzung gemäß Anspruch 13, wobei das Galactolipid aus fraktioniertem Haferöl besteht.

15. Zusammensetzung gemäß Anspruch 14, wobei die Menge an fraktioniertem Haferöl etwa 1,5 Gew.-% der gesamten Zusammensetzung bildet.

16. Zusammensetzung gemäß Anspruch 1 in der Form einer Öl-in-Wasser-Emulsion.

17. Zusammensetzung gemäß einem der Ansprüche 1 bis 16 zur Verwendung bei der Therapie.

18. Verwendung der Zusammensetzung gemäß einem der Ansprüche 1 bis 16 bei der Herstellung eines Medikaments zur Behandlung oder Vorbeugung von Obesitas.

19. Nahrungsmittelzusatzstoff, im Wesentlichen bestehend aus einem Triacylglycerin oder einem Gemisch von Triacylglycerinen, deren Acylreste ausgewählt sind aus der Gruppe, bestehend aus Tetradecanoyl bis Eicosanoyl, gegebenenfalls einfach oder zweifach ungesättigt, mit der Maßgabe, dass mehr als 15 mol-% aller Acylreste aus α-Alkyl-substituierten Acylresten bestehen, wobei die α-Alkyl-Substituenten bis zu 9 Kohlenstoffatome umfassen, einem Nahrungsmittel-Emulgator und gegebenenfalls einem oder mehreren von Wasser, Natriumchlorid, Antioxidationsmittel, Farbmittel, Aromastoff.

20. Nahrungsmittelzusatzstoff gemäß Anspruch 19, wobei der Nahrungsmittel-Emulgator aus Lecithin und Galactolipid ausgewählt ist.

21. Nahrungsmittelprodukt, umfassend eine Obesitasverhindernde Menge des Nahrungsmittelzusatzstoffs gemäß Anspruch 19 oder 20.

22. Nahrungsmittelprodukt gemäß Anspruch 21 in der Form eines Milchprodukts.

23. Milchprodukt gemäß Anspruch 22 in der Form von Joghurt, Sahne, saurer Sahne, Milch, Butter oder einem Butterersatz, der Milchfett umfasst, Eiscreme.

24. Nahrungsmittelprodukt gemäß Anspruch 21 in der Form eines Nicht-Milchprodukts.

25. Nahrungsmittelprodukt gemäß Anspruch 21, wobei mehr als 50 mol-% des Triacylglyceringemischs 1-(2-Methyloctadecanoyl)-2,3-dihexadecanoylglycerin sind.

26. Nahrungsmittelprodukt gemäß Anspruch 21, wobei etwa ein Drittel der Acylreste Octadecanoyl sind und etwa zwei Drittel 1-(2-Methyloctadecanoyl) sind.

## Revendications

1. Composition, en particulier composition alimentaire comprenant une quantité satiétogène d'un triacylglycérol ou d'un mélange de triacylglycérols, dont les résidus acyle sont choisis dans le groupe constitué de tétradécanoyle à eicosanoyle, éventuellement mono- ou di-insaturés, à condition que plus de 15 % en moles des résidus acyle totaux soient constitués de résidus acyle substitués par alkyle en α, les substituants alkyle en α comprenant jusqu'à 9 atomes de carbone, et un émulsifiant alimentaire.

2. Composition selon la revendication 1, **caractérisée en ce que** plus de 50 % en moles desdits résidus acyle substitués en α comprennent un membre du groupe constitué de 2-méthyltétradécanoyle, 2-méthylhexadécanoyle, 2-méthyloctadécanoyle, 2-méthyleicosanoyle, et des mélanges de ceux-ci.

3. Composition selon la revendication 1, **caractérisée en ce que** plus de 80 % en moles desdits résidus acyle substitués en α comprennent un membre du groupe constitué de 2-méthyltétradécanoyle, 2-méthylhexadécanoyle, 2-méthyloctadécanoyle, 2-méthyleicosanoyle, et des mélanges de ceux-ci.

4. Composition selon la revendication 1, **caractérisée en ce que** plus de 90 % en moles desdits résidus acyle substitués en α comprennent un membre du groupe constitué de 2-méthyltétradécanoyle, 2-méthylhexadécanoyle, 2-méthyloctadécanoyle, 2-méthyleicosanoyle, et des mélanges de ceux-ci.

5. Composition selon la revendication 1, **caractérisée en ce que** plus de 95 % en moles desdits résidus acyle substitués en α comprennent un membre du groupe constitué de 2-méthyltétradécanoyle, 2-méthylhexadécanoyle, 2-méthyloctadécanoyle, 2-méthyleicosanoyle, et des mélanges de ceux-ci.

6. Composition selon l'une quelconque des revendications 1 à 5, **caractérisée en ce que** ledit résidu acyle substitué en α est 2-méthyloctadécanoyle.

7. Composition selon la revendication 6, **caractérisée en ce que** lesdits résidus acyle qui ne sont pas substitués en α sont choisis parmi hexadécanoyle et octadécanoyle.

8. Composition selon la revendication 1, **caractérisée en ce que** lesdits résidus acyle comprennent hexadécanoyle et 2-méthyloctadécanoyle, à condition que 15 % en moles des résidus acyle ou plus soient des résidus 2-méthyloctadécanoyle.

9. Composition selon la revendication 8, **caractérisée en ce que** lesdits résidus acyle sont essentiellement constitués d'hexadécanoyle et de 2-méthyloctadécanoyle.

10. Composition selon la revendication 8 ou 9, **caractérisée en ce que** le rapport molaire d'hexadécanoyle sur 2-méthyloctadécanoyle est environ 1:2.

11. Composition selon la revendication 1, constituée essentiellement de tripalmitine, de tri(2-méthylstéaroyl)glycérol, de 1,2-di(2-méthylstéaroyl)-3-palmitoylglycérol, de 1,3-di(2-méthylstéaroyl)-2-palmitoylglycérol, de 1-(2-méthylstéaroyl)-2,3-dipalmitoylglycérol, et de 1,3-dipalmitoyl-2-(2-méthylstéaroyl)glycérol.

12. Composition selon la revendication 1, **caractérisée en ce que** l'émulsifiant alimentaire comprend la lécithine.

13. Composition selon la revendication 1, **caractérisée en ce que** l'émulsifiant alimentaire comprend le galactolipide.

14. Composition selon la revendication 13, **caractérisée en ce que** le galactolipide comprend de l'huile d'avoine fractionnée.

15. Composition selon la revendication 14, **caractérisée en ce que** la quantité d'huile d'avoine fractionnée est d'environ 1,5 % en poids de la composition totale.

16. Composition selon la revendication 1, sous forme d'émulsion huile dans l'eau.

17. Composition selon l'une quelconque des revendications 1 à 16, destinée à être utilisée en thérapie.

18. Utilisation de la composition selon l'une quelconque des revendications 1 à 16, dans la fabrication d'un médicament destiné au traitement ou à 1a prévention d'obésité.

19. Additif alimentaire constitué essentiellement d'un triacylglycérol ou d'un mélange de triacylglycérols, dont les résidus acyle sont choisis dans le groupe constitué de tétradécanoyle à eicosanoyle, éventuellement mono- ou di-insaturés, à condition que plus de 15 % en moles des résidus acyle totaux soient constitués de résidus acyle substitués par alkyle en α, les substituants alkyle en α comprenant jusqu'à 9 atomes de carbone, un émulsifiant alimentaire et, éventuellement, un ou plusieurs parmi l'eau, le chlorure de sodium, un antioxydant, un colorant et un arome.

20. Additif alimentaire selon la revendication 19, **caractérisé en ce que** ledit émulsifiant alimentaire est choisi parmi la lécithine et le galactolipide.

21. Produit alimentaire comprenant une quantité anti-obésité de l'additif alimentaire selon la revendication 19 ou 20.

22. Produit alimentaire selon la revendication 21, sous forme de produit laitier.

23. Produit laitier selon la revendication 22, sous forme de yaourt, de crème, de crème fermentée, de lait, de beurre ou d'un substitut de beurre comprenant de la graisse de lait, de glace.

24. Produit alimentaire selon la revendication 21, sous forme de produit non laitier.

25. Produit alimentaire selon la revendication 21, **caractérisé en ce que** plus de 50 % en moles dudit mélange de triacylglycérols est le 1-(2-méthyloctadécanoyl)-2,3-dihexadécanoyl-glycérol.

26. Produit alimentaire selon la revendication 21, **caractérisé en ce qu'**environ un tiers desdits résidus acyle sont octadécanoyle et environ deux tiers sont 1-(2-méthyloctadécanoyle).
